# EUROPEAN PATENT SPECIFICATION

(11) **EP 2 089 089 B1**
(45) Date of publication and mention of the grant of the patent: **04.08.2010**
(21) Application number: 07862395.6
(22) Date of filing: 30.11.2007
(51) Int. Cl.: A61M 25/01, A61F 2/84

(54) **Pusher sheath and deployment device**
Schubschlauch für Stent-Implantationsvorrichtung
Gaine pour pousser un implant et dispositif de déploiement

(30) Priority: 30.11.2006 US 861868 P
(43) Date of publication of application: 19.08.2009
(73) Proprietor: William Cook Europe ApS, 4632 Bjaeverskov (DK); Cook Incorporated, Bloomington, IN 47402-0489 (US)
(72) Inventor: RASMUSSEN, Erik E., 4200 Slagelse (DK)
(74) Representative: Jehan, Robert
(86) International application number: PCT/US2007/024685
(87) International publication number: WO 2008/066917

(56) References cited:
- WO-A-00/72780
- WO-A-2004/080504
- US-A1- 2001 001 833
- US-A1- 2005 049 609
- US-A1- 2005 085 891

## Description

### Technical Field

The present invention relates to a pusher sheath or pusher rod and deployment device including such a pusher sheath or rod. The pusher sheath is intended for use in delivering stents, stent grafts and other devices typically delivered by percutaneous delivery methods well known in the art.

### Background of the Invention

Deployment devices for deploying stents, stent grafts and other intra luminal devices typically include: a guidewire, a guidewire catheter, an insert support (which may be a portion of the guidewire catheter), a pusher sheath or rod, a proximal manipulation unit, as well as various other components well known in the art. The guidewire, which is typically provided with radio opaque markings or other markers, is typically made of a very flexible metal or metallic material and is introduced before the other components of the deployment device. For example, for the deployment of a stent or stent graft into the aortic artery, the guidewire can be passed percutaneously from the femoral artery, for example. Once the guidewire is in place, the distal part of the deployment device, that is the unit of guidewire catheter, device to be deployed and pusher sheath or rod, is then inserted so as to follow the guidewire. Once in position, the implant can be released from the deployment device, which deployment device is then removed from the patient in a conventional manner.

The guidewire catheter is provided with a flexible dilator tip at its distal end for dilating the passages, percutaneous and luminal, for receipt of the deployment device and thus of the implant. In order to provide flexibility at the distal end of the deployment device, the guidewire catheter on which the implant is located during the deployment operation is flexible so as to be able to follow any tortuous path previously followed by the guidewire. On the other hand, the pusher sheath or rod (sometimes known as an inner sheath) needs to be significantly stiffer than the guidewire catheter in order to provide the pushing force required to push the distal end of the deployment device and the implant to the deployment site. Typically, the pusher sheath is significantly more rigid than the other components of the deployment device and implant, even than the outer sheath which holds the implant in place until it reaches the deployment site.

Conventional deployment assemblies of this type generally work satisfactorily. However, problems can arise when the path to be followed by the deployment device is very tortuous. In some instances, it is possible for the guidewire catheter to kink at its junction with the pusher sheath, with the result that the deployment device snags as the transition from the relatively flexible guidewire catheter to the relatively rigid pusher sheath reaches this tortuous point in the vessel. This can make the device useless, thus forcing the medical procedure to be aborted before its completion, with consequential trauma to the patient and delay in carrying out the procedure.

WO-2004/080504 discloses a device for reducing the effect of such bending or kinking. This document discloses the provision of a tubular insert which is located between the distal end of the pusher rod and the stent or stent graft to be implanted. The tubular insert includes a tube of flexible material having at either end an enlarged member. Adjacent the distal end of the pusher sheath, the enlarged member may be made of metal or a plastics material. The enlarged end member adjacent the stent or stent graft, on the other hand, is made of a relatively soft material, such as a low density polymer. This relatively soft distal member is intended to receive preload pressure of the stent or stent graft and also to push the stent graft out of the introducer catheter at the moment of deployment. The tubular insert is of a length sufficient to accommodate the entire expected length of the tortuous path of a patient's vessel through which the device is intended to pass. As explained, the pusher end of this tubular member acts both to push the stent or stent graft and also to absorb some of the force produced by that stent or stent graft during its preloading and deployment.

The problem of kinking is also addressed in other ways, for example as disclosed In US-2005/0049609 and EP-A-1,208,816. In the case of the devices disclosed in these documents, the problem of kinking is generally addressed by providing a flexible coil insert between the guidewire catheter and the outer sheath of the deployment device. The insert thus adds solidity to the device by providing a barrier against inward movement of the outer sheath of the deployment device. US2001/0001833 disposes a device to assist delivery and positioning by providing elongate members extending through openings In a prosthesis to maintain the position of the prosthesis on the catheter.

### Summary of the Invention

The present invention seeks to provide an Improved deployment device and an Improved pusher member for such a deployment device.

According to an aspect of the present invention as claimed in claim 1, there Is provided a pusher member for a deployment device having a generally cylindrical tubular form with a channel therein for receiving a guide wire, the pusher member including a proximal portion and a distal portion, wherein the distal end of the pusher member has a reduced outer diameter.

Preferably, the pusher member has a major portion which has a substantially uniform outer diameter.

The reduced outer diameter portion reduces the bending stiffness of the pusher member to give the pusher member more flexibility at its distal end. It has been found that this reduction in outer diameter, which in effect reduces the radial thickness of the wall of the member at this location, does not significantly affect its longitudinal stiffness, thereby not significantly affecting the efficacy of the pusher member.

Advantageously, the reduced outer diameter portion of the pusher member ends short of the distal end itself, thereby leaving a pusher head against which a stent or stent graft fitted to the deployment device can abut. This pusher head is preferably of a longitudinal dimension sufficient to provide a substantially equivalent rigidity in the longitudinal direction as a conventional pusher member which has no reduced outer diameter section.

In the preferred embodiment, the reduced outer diameter section of the pusher member tapers towards the distal end thereof. It is preferred that this taper extends over a substantial distance, for example up to around 200 mm and in preferred embodiments up to around 150 mm. The advantage of having a reduced diameter section of this length is that it provides a substantial longitudinal extent of pusher member which has an increased flexibility compared the remainder of the pusher member and in practice of conventional pusher members. This assists movement of the delivery device around the tortuous paths of a patient's lumens. In the case where the reduced outer diameter section tapers towards the distal end, this produces a gradually increasing stiffness from the distal end towards the proximal end of the pusher member, which further facilitates the motion of the deployment device through a patient.

In some embodiments, the reduced outer diameter section of the pusher member may be partially tapering and partially cylindrical.

The pusher head of the pusher member may be provided with one or more bores for receipt of a trigger and/or release wire of the deployment device.

According to another aspect of the present invention, there is provided a deployment device including a pusher member as herein specified.

### Brief Description of the Drawings

Embodiments of the present invention are described below, by way of example only, with reference to the accompanying drawings, in which:
Figures 1 and 2 show an embodiment of a known deployment device;
Figure 3 shows in cross-section the distal end of a prior art deployment device;
Figure 4 shows in cross-section the distal end of an embodiment of deployment device;
Figure 5 is a side elevational view of an embodiment of pusher, member;
Figure 6 is a longitudinal cross-sectional view of the pusher member of Figure 5;
Figure 7 is a cross-sectional view of the distal end of the embodiment of pusher member of Figure 5;
Figure 8 is a longitudinal view of a second embodiment of pusher member;
Figure 9 is a longitudinal cross-sectional view of the embodiment of pusher member of Figure 8;
Figure 10 is a cross-sectional view of the pusher head of the embodiment of pusher member of Figure 8;
Figure 11 is a cross-sectional view of another embodiment of pusher member; and
Figure 12 is an enlarged view of the distal end of the pusher member of Figure 11.

### Detailed Description

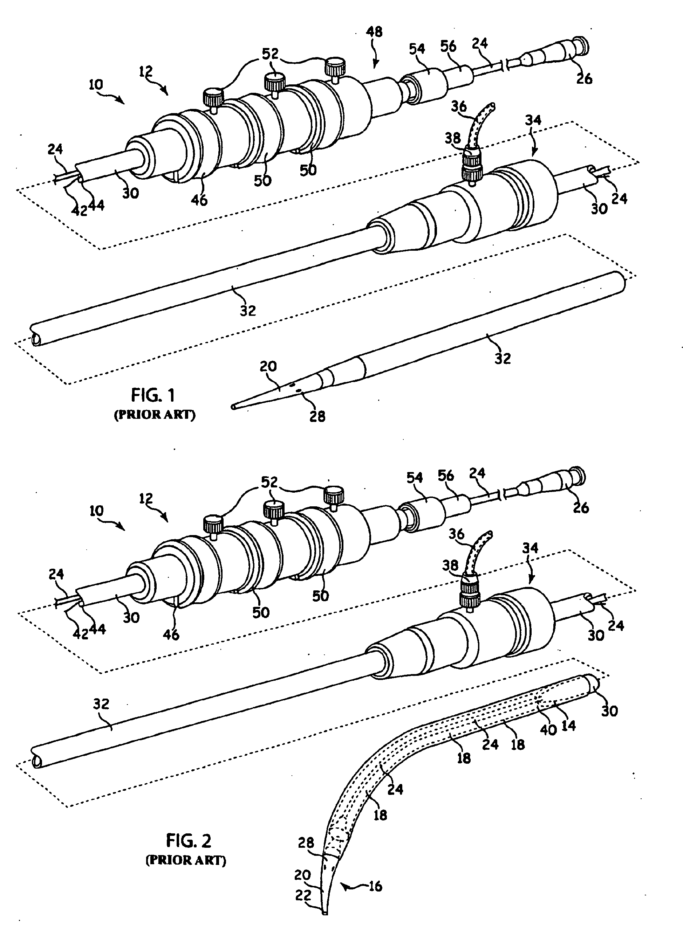

Referring to Figures 1 and 2, the introducer 10 includes an external manipulation section 12, a distal attachment region 14 and a proximal attachment region 16. The distal attachment region 14 and the proximal attachment region 16 secure the distal and proximal ends of the implant 18, respectively. During the medical procedure to deploy the implant 18, the distal and proximal attachment regions 14 and 16 will travel through the patient's lumen to a desired deployment site. The external manipulation section 12, which is acted upon by a surgeon to manipulate the introducer, remains outside of the patient throughout the procedure.

The proximal attachment region 16 of the introducer 10 includes a dilator tip 20, which is typically provided with a bore 22 therein for receiving a guide wire (not shown) of conventional type. The longitudinal bore 22 also provides a channel for the introduction of medical reagents. For example, it may be desirable to supply a contrast agent to allow angiography to be performed during placement and deployment phases of the medical procedure.

A guide wire catheter 24, conventionally made from a flexible thin walled metal tube, is fastened to the dilator tip 20. The guide wire catheter 24 is flexible so that the introducer 10 can be advanced along a relatively tortuous vessel, such as a femoral artery, and so that the distal attachment region 14 can be longitudinally and rotationally manipulated. The guide wire catheter 24 extends through the introducer 10 to the manipulation section 12, terminating at a connection device 26, in conventional manner.

The connection device 26 is designed to accept a syringe to facilitate the introduction of reagents into the inner catheter 24. The guide wire catheter 24 is in fluid communication with apertures 28 in the flexible dilator tip 20. Therefore, reagents introduced into connection device 26 will flow to and emanate from the apertures 28.

A pusher sheath or rod 30 (hereinafter referred to as a pusher member), typically made from a plastics material, is mounted coaxial with and radially outside of the guide wire catheter 24. The pusher member 30 is "thick walled", that is the thickness of its wall is preferably several times greater than that of the guide wire catheter 24.

A sheath 32 extends coaxially over and radially outside of the pusher member 30. The pusher member 30 and the sheath 32 extend distally to the manipulation region 12.

The implant 18, which may be a stent, a stent-graft or any other implant or prosthesis deliverable by this device 10, is retained in a compressed condition by the sheath 32. The sheath 32 extends distally to a sheath manipulator and haemostatic sealing unit 34 of the external manipulation section 12. The haemostatic sealing unit 34 includes a haemostatic seal (not shown) and a side tube 36 held to the unit 34 by a conventional luer lock 38.

The sheath manipulator and haemostatic sealing unit 34 also includes a clamping collar (not shown) that clamps the sheath 32 to the haemostatic seal and a silicone seal ring (not shown) that forms a haemostatic seal around the pusher rod 30. The side tube 38 facilitates the introduction of medical fluids between the pusher rod 30 and the sheath 32. Saline solution is typically used.

During assembly of the introducer 10, the sheath 32 is advanced over the proximal end of the dilator tip 20 of the proximal attachment region 16 while the implant 18 is held in a compressed state by an external force. A suitable distal attachment (retention) section (not visible in this view) is coupled to the pusher rod 30 and retains a distal end 40 of the prosthesis 18 during the procedure. The distal end of the prosthesis 18 is provided with a loop (not shown) through which a distal trigger wire 42 extends. The distal wire also extends through an aperture (not shown in Figures 1 and 2) in the distal attachment section 40 into an annular region 44 between the inner catheter 24 and the pusher rod 30. The distal trigger wire 42 extends through the annular space 44 to the manipulation region 12 and exits the annular space 44 at a distal wire release mechanism 46.

A proximal portion of the external manipulation, section 12 includes at least one release wire actuation section 50 mounted on a body 48, in turn mounted onto the pusher member 30. The guidewire catheter 24 passes through the body 48. The distal wire release mechanism 46 and the proximal wire release mechanism 50 are mounted for slidable movement on the body 48.

The positioning of the proximal and distal wire release mechanisms 46 and 50 is such that the proximal wire release mechanism 46 must be moved before the distal wire release mechanism or mechanisms 50 can be moved. Therefore, the distal end of the implant 18 cannot be released until a self-expanding zigzag stent thereof has been released. Clamping screws 52 prevent inadvertent early release of the prosthesis 18. A haemostatic seal (not shown) is included so that the release wires can extend out through the body 48 without unnecessary blood loss during the medical procedure.

A proximal portion of the external manipulation section 12 includes a pin vice 54 mounted onto the proximal end of the body 48. The pin vice 54 has a screw cap 56. When screwed in, vice jaws (not shown) of the pin vice 54 clamp against or engage the guidewire catheter 24. When the vice jaws are engaged, the guidewire catheter 24 can only move with the body 48 and hence it can only move with the pusher member 30. With the screw cap 56 tightened, the entire assembly can be moved together as one piece.

Once the introducer assembly 12 is in the desired deployment position, the sheath 32 is withdrawn to just proximal of the distal attachment section 14. This action releases the middle portion of the implant 18, in this example a stent or stent-graft, so that it can expand radially. Consequently, the stent or stent-graft 18 can still be rotated or lengthened or shortened for accurate positioning. The proximal end self-expanding stent however, is still retained at the dilator tip 16 by means of the release wires. Also, the distal end of the stent or stent-graft 18 will still retained within the sheath 32.

Next, the pin vice 54 is released to allow small movements of the guidewire catheter 24 with respect to the pusher rod 30 to allow the stent or stent-graft 18 to be lengthened, shortened, rotated or compressed for accurate placement in the desired location within the lumen. X-ray opaque markers (not shown) may be placed along the stent or stent-graft 18 to assist with placement of the prosthesis.

When the proximal end of the stent or stent-graft 18 is in place, the proximal trigger wire is withdrawn by distal movement of the proximal wire release mechanism. The proximal wire release mechanism 50 and the proximal trigger wire can be completely removed by passing the proximal wire release mechanism 50 over the pin vice 54, the screw cap 56 and the connection unit 26.

Next, the screw cap 56 of the pin vice 54 is loosened, after which the inner catheter 24 can be pushed in a proximal direction to move the dilator tip 20 in a proximal direction. When the dilator tip 20 no longer surrounds the end of the stent or stent-graft 18, it expands to engage the lumen walls of the patient. From this stage on, the proximal end of the stent or stent-graft 18 cannot be moved again.

Once the proximal end of the stent or stent-graft 18 is anchored, the sheath 32 is withdrawn distally of the distal attachment section 14, which withdrawal allows the distal end of the stent or stent-graft 18 to expand. At this point, the distal end of the stent or stent-graft 18 may still be repositioned as needed.

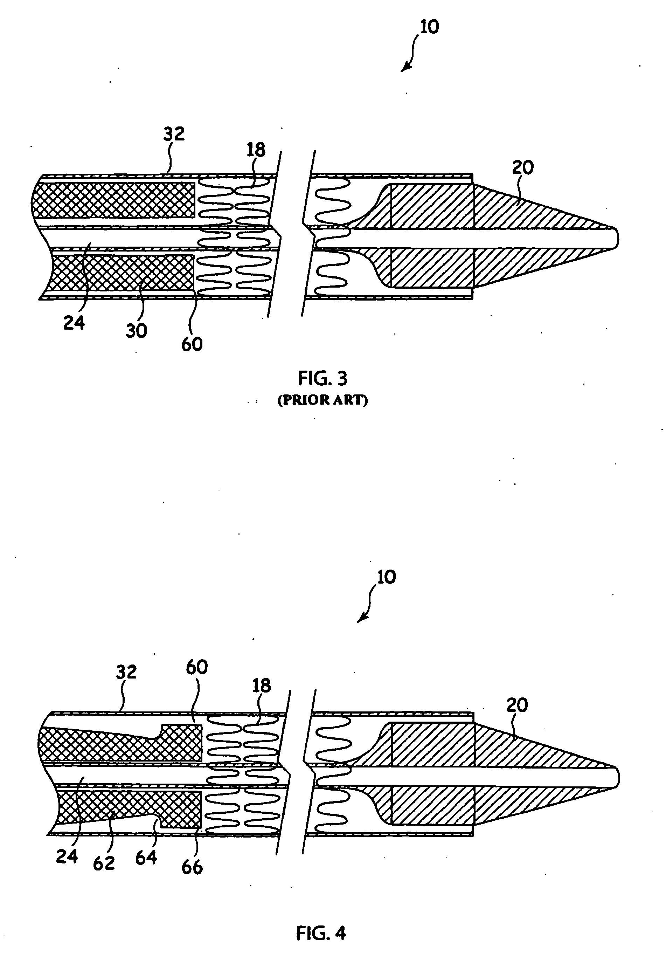

Figure 3 shows in enlarged form a cross-sectional view of the distal portion of the deployment device. The Figure is in schematic form for ease of understanding.

As can be seen, a stent 18 is retained in compressed form between the flexible dilator tip 20 and pusher member 30 around the guidewire catheter 24 and held in this compressed state at least in part by the outer sheath 32. In practice there are also provided release wires (not shown in Figure 3) for retaining the ends of the stent 18. These are of conventional type and therefore are not described herein in further detail.

The pusher member 30 is of substantially uniform thickness, as is conventional with pusher members of this type. At the junction 60 between the distal end of the pusher member 30 and the guidewire catheter 24, there is an abrupt transition in bending resiliency, caused as a result of the different bending stiffnesses of the pusher member 30 and the guidewire catheter 24. As a result of this abrupt transition in bending stiffnesses, it is possible for the guidewire catheter 24 to kink at this location 60, causing potential failure of the deployment device during the cause of deployment.

Figure 4 shows a similar view of a first embodiment of deployment device 10'. The components of the deployment device 10' are generally the same as those of the deployment device 10 of Figures 1 to 3. In other words, it includes the flexible dilator 20, a guidewire catheter 24 and an outer sheath 32. It can deliver a stent or stent-graft 18 or any other implant as any deployment device of this nature. The pusher member 62 differs from the pusher member 30 of the examples of Figures 1 to 3 in that it is provided with a portion 64 which has a reduced outer circumference. As can be seen from Figure 4, this portion 64 of reduced outer circumference reduces the thickness of the wall of the pusher member 62.

The very end of the pusher member 62 is provided with a pusher head 66 which has an outer diameter preferably the same as the outer diameter of the major portion of the pusher member 62. In effect, the region of reduced outer diameter or circumference can be produced by machining or etching the outer surface of the pusher member 62 at the location where it is desired to reduce its diameter.

As a result of the reduced thickness of the pusher member walls in this region, the pusher member 62 is more flexible at this section. This has the effect of reducing substantially the difference in flexibilities between the pusher member 62 and the guidewire catheter 24 at the junction 60 and therefore reduces substantially the possibility of kinking of the device at the junction 60.

The pusher head 66 retains the characteristics of any such pusher member, that is it has a longitudinal stiffness comparable with that of a conventional pusher member such as that disclosed in the examples of Figures 1 to 3. For this purpose, the longitudinal thickness of the pusher head 66 is preferably such as to keep it relatively rigid and of a rigidity equivalent to the rigidity of a conventional cylindrical pusher member.

The reduced outer diameter portion 64 of this embodiment is rounded at its transition to the pusher head 66, which has the advantage of avoiding abrupt transitions in one of the surfaces of the pusher member 62. This can avoid potential failures of the component and can also avoid snagging of other components of the deployment device.

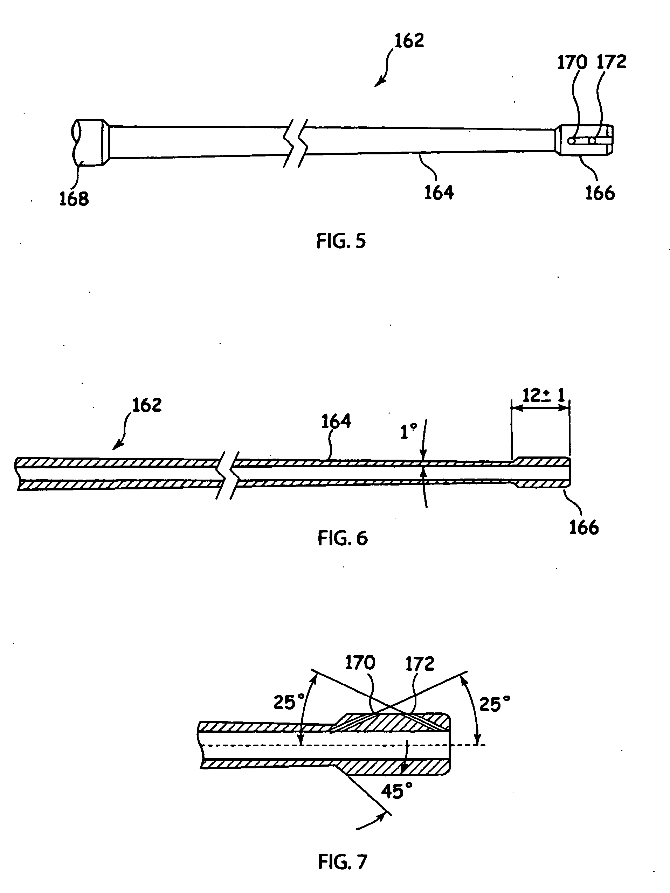

The embodiment of pusher member 62 shown in Figure 4 also has a reduced diameter section which tapers towards the distal end of the pusher member 62. This characteristic is explained and described in further detail below in connection with the embodiments of Figures 5 to 10.

Referring now to Figure 5, an embodiment pusher member 162 has a reduced outer diameter section 164 which is located between the main portion 168 of the pusher member 162 and a pusher head 166, in similar manner to the embodiment of Figure 4. In this embodiment, the reduced diameter section 164 has a maximum diameter which is smaller than the outer diameter of the main section 168 of the pusher member, as can be seen to the left-hand side of Figure 5. The portion 164 tapers towards the pusher head 162.

For a 7,33 mm (22 French) pusher member the length of the reduced diameter section 164 may be in the region of 70 to 80 mm, preferably around 75 mm. As can be seen in particular in Figure 6, the pusher member has an internal bore with a diameter of around 3.5 mm (plus or minus 0.1 mm) for receiving a guidewire catheter 24, in conventional manner. Figure 6 also shows the reduced diameter portion 164 having a taper of one degree from the distal end towards the proximal end of the pusher member 162.

In this particular example, the pusher member 162 has an outer diameter of 11 mm plus or minus 1 mm for the section 168; the maximum outer diameter of the reduced diameter portion 164 is 8 mm plus or minus 1 mm and its smallest outer diameter is 5 mm plus or minus 1 mm. The pusher head 166 has an outer diameter, in this embodiment, of 8 mm plus or minus 1 mm and a length of 12 mm plus or minus 1 mm.

The pusher head 166 is provided with first and second bores 170, 172 which in this example, as shown in Figure 7, extend at an angle of around 25° to the axis of the pusher member 162. These bores allow for a trigger wire or release wire to pass from the inside of the pusher member 162 and eventually to be coupled to one of the ends of the stent or stent-graft held within the deployment device. As can be seen in particular in Figure 5, a small channel is provided in the outer surface of the pusher 166, so that such a trigger wire can be held within the perimeter of the pusher head 166 and so as not to snag against the inner surface of the sheath 32 of the deployment device.

For a 6,66 mm (20 French) pusher member 162, the length of the tapered portion would typically be in the region of 52 to 62 mm, preferably around 57 mm and the length of the pusher head 166 would be correspondingly reduced, for example by around half a millimetre compared to a 7,33 mm (22 French) pusher member 162.

The stent 18 would abut against the distal end of the pusher head 166, in a similar manner as shown in Figure 4.

It will be appreciated that the reduced diameter section 164 of the pusher member 162 is of a substantial length compared to the expected length of the most tortuous part of a patient's vessel. The length and taper of this reduced diameter section 164 provides for a gradual transition in stiffness of the pusher member and therefore of the assembly of guidewire catheter 24 with stent 18 thereon and pusher member 162. Thus, the device stiffens gradually from its distal end to its proximal end. This can significantly reduce the chances of kinking of the assembly, thereby significantly reducing the chances of an abortive deployment procedure. It also enables the device to bend around more tortuous vessels than prior art assemblies.

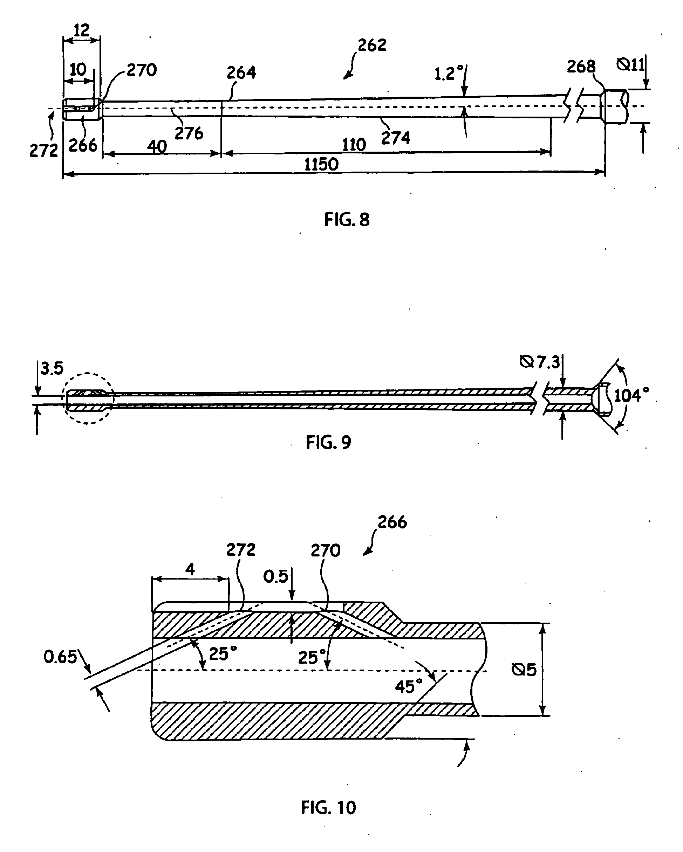

Figures 8 to 10 disclose another embodiment of pusher member 262. The pusher member 262 is similar to the pusher member 162 shown in Figures 5 to 7, as is the pusher head 266 to the pusher head 166. The principal difference between these two embodiments is that the reduced diameter section 264 of the pusher member 262 has a first portion 274 which tapers from the main portion 268, in this example at a taper angle of 1.2 degrees, as well as a second reduced diameter portion 276 which is substantially cylindrical. The tapering portion 274, in this example, has a length of around 110 mm, while the cylindrical portion 276 has a length of 40 mm. This example is for a pusher member 262 of 7,33 mm (22 French).

For a 6,66 mm (20 French) pusher member the length of the tapering portion 274 would preferably be in the region of around 80 mm.

Once again, the minimum outer diameter of the reduced diameter section 264 is preferably in the region of 5 mm plus or minus 1 mm, while its largest diameter is preferably around 8 mm plus or minus 1 mm. The dimensions of the pusher head 266 are equivalent to the dimensions of the pusher head of 166 of the embodiment of Figures 5 to 7.

It will be appreciated by the skilled person that the pusher member 262 of the embodiment of Figures 8 to 10 will have a first portion, that is portion 276, which has a relatively uniform higher flexibility and a second section, the tapering section 274, which has an increasing stiffness towards the main portion 268 of the pusher member 262. In some instances it may be desirable to lengthen the proportion of the pusher member which has an increased flexibility, for which it is inappropriate to have the entire length of the reduced diameter portion tapering as this could lead to an angle of taper which is too shallow.

The various dimensions given in connection with the embodiments of Figures 5 to 10 are given solely by way of example. The person skilled in the art will appreciate that such pusher members are provided in varying dimensions and characteristics, in dependence upon the characteristics of the deployment device into which they are to be used and the application in which the deployment device is to be used, such as the nature of the patient or the nature of the implant to be fitted. These dimensions therefore are not to be taken as limiting in any way.

The embodiments of pusher member disclosed above include a tapering or part-tapering reduced outer diameter portion. This taper is a linear taper but this is not necessary in many applications. Other tapers could be used, for example a curved or rounded taper. In some instances the reduced outer diameter portion could be formed of a plurality of sections of different outer diameters to produce different flexibilities at each of the sections. A tapering section is preferred, however, as this provides a gradual change in stiffness of the pusher member and therefore of that part of the deployment device.

The pusher member shown in the embodiments of Figures 4 to 10 is preferably made of the same material as existing pusher members.

In some instances, release wires are not used to release a stent or stent-graft held within the deployment device, in which case it is not necessary to make provision for the release wires within the pusher head 66, 166, 266 of the pusher member. In such a circumstance, the length of the pusher head can be reduced compared to the lengths shown in the Figures and described above. It can be reduced, preferably, to an extent whereby it remains relatively stiff in the longitudinal direction so as to provide a good abutment shoulder against which a stent or stent-graft can press, with no noticeable deformation of the pusher head compared to the distal end of a conventional pusher member.

It is also envisaged in applications in which a stent or stent-graft or other implant held within the deployment device is pulled with the device rather than being pushed by the device, that the pusher head 66, 166, 266 could be omitted in its entirety. In this case, the end of the pusher member would simply taper and retain a narrow cylindrical end portion, such as the reduced outer diameter section 262 of the embodiment of Figure 8.

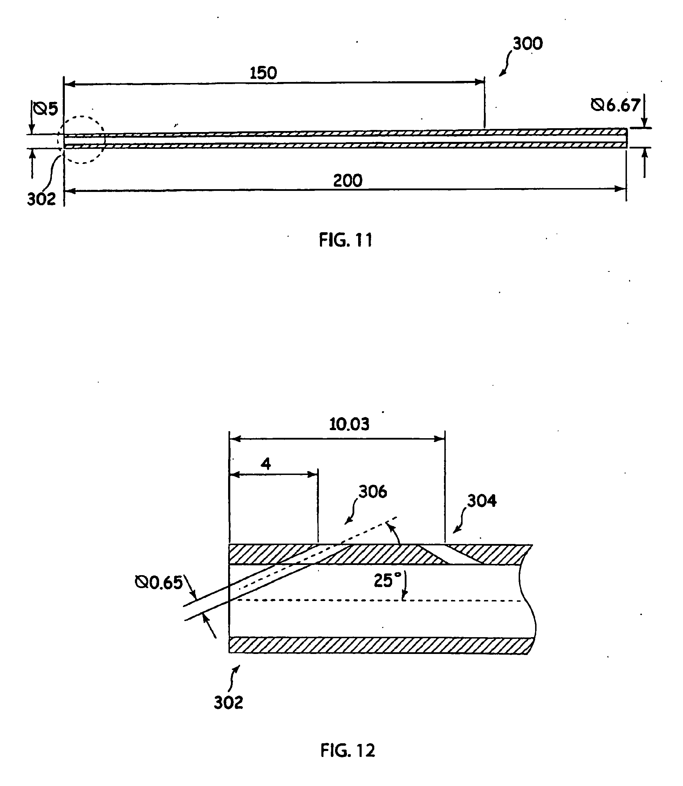

An example of this can be seen in Figures 11 and 12. The embodiment of Figure 11 includes a member 300 which tapers towards its distal end 302. In this example, the overall length of the tapered portion is in the region of 200mm, although the significant reduction in radial stiffness occurs in the last 150mm of the member 300. At the start of the taper, the outer diameter of the member 300 is in the region of 6.67mm (in this example), whilst its distal end has an outer diameter of around 5mm.

Referring in particular to Figure 12, the distal end 302 of the member 300 is provided with a plurality of restraining wire fixing points 304, 306 which are oriented relative to one another when looking towards the outer surface of the end 302, such that a restraining wire can be fed from the inside of the member 300 through the location 304 and then looped back into the location 306. Such looping of the restraining wires will keep these in place until they are positively withdrawn by a suitable withdrawal mechanism, such as that shown in connection with Figures 1 and 2.

In such a circumstance, the reduced outer diameter section of the pusher member could be considered a tapered stiffener for the deployment device as the member would not actually have the function of pushing the implant to its deployment position. It will instead simply act as an element which varies the stiffness of the deployment device, in order to achieve the advantages described and taught herein.

## Claims

1. A pusher member (62) for a deployment device (10) having a generally cylindrical tubular form with a channel therein for receiving a guide wire, the pusher member (62) being provided with a proximal end and a distal end, wherein the distal end of the pusher member (62) has a reduced outer diameter section (64) which tapers towards the distal end thereof **characterized by** an angle of taper with respect to the longitudinal axis of the pusher member being no more than 2°.

2. A pusher member according to claim 1, wherein a major portion of the pusher member (62) has a substantially uniform outer diameter.

3. A pusher member according to claim 1 or 2, including a pusher head (66) between the reduced outer diameter portion (64) and the distal end of the pusher member.

4. A pusher member according to claim 3, wherein the pusher head (66) is provided with one or more bores (170, 172) for receipt of a trigger and/or release wire of a deployment device.

5. A pusher member according to any preceding claim, wherein said reduced outer diameter section (64) extends over a distance of up to around 200 mm.

6. A pusher member according to any preceding claim, wherein said reduced outer diameter section (64) extends over a distance of up to around 150 mm.

7. A pusher member according to any preceding claim, wherein the length of the reduced diameter section (64) is between about 70 and about 80 mm.

8. A pusher member according to any preceding claim, wherein the or a maximum outer diameter of the reduced outer diameter section (64) is 8 mm ±1 mm.

9. A pusher member according to any preceding claim, wherein the or a minimum outer diameter of the reduced outer diameter section (64) is 5 mm ±1 mm.

10. A pusher member according to any preceding claim, wherein the tapering portion has at least one of the following characteristics: it is a linear
taper, a curved taper and a rounded taper.

11. A pusher member according to any preceding claim, wherein the reduced outer diameter section (64) is at least partially cylindrical.

12. A pusher member according to any preceding claim, wherein the pusher member (62) includes a bore (170, 172) therethrough.

13. A pusher member according to claim 12, wherein the bore (170, 172) of the pusher member (62) has a smooth and substantially uniform inner wall.

14. A deployment device Including a pusher member (62) according to any preceding claim.

## Patentansprüche

1. Schubelement (62) für eine Implantationsvorrichtung (10) mit einer allgemein zylindrischen röhrenförmigen Gestalt mit einem Kanal darin zur Aufnahme eines Führungsdrahts, wobei das Schubelement (62) mit einem proximalen Ende und einem distalen Ende versehen ist, wobei das distale Ende des Schubelements (62) einen Abschnitt (64) mit verringertem Außendurchmesser aufweist, der sich zu seinem distalen Ende hin verjüngt, **gekennzeichnet durch** einen Verjüngungswinkel relativ zur Längsachse des Schubelements von höchstens 2°.

2. Schubelement nach Anspruch 1, worin ein Hauptteil des Schubelements (62) einen im Wesentlichen gleichmäßigen Außendurchmesser aufweist.

3. Schubelement nach Anspruch 1 oder 2, mit einem Schubkopf (66) zwischen dem Abschnitt (64) mit verringertem Außendurchmesser und dem distalen Ende des Schubelements.

4. Schubelement nach Anspruch 3, worin der Schubkopf (66) mit einer oder mehr Bohrungen (170, 172) zur Aufnahme eines Trigger- und/oder Freigabedrahts einer Implantationsvorrichtung versehen ist.

5. Schubelement nach einem der vorhergehenden Ansprüche, worin sich der Abschnitt (64) mit verringertem Außendurchmesser über eine Strecke von bis zu ca. 200 mm erstreckt.

6. Schubelement nach einem der vorhergehenden Ansprüche, worin sich der Abschnitt (64) mit verringertem Außendurchmesser über eine Strecke von bis zu ca. 150 mm erstreckt.

7. Schubelement nach einem der vorhergehenden Ansprüche, worin die Länge des Abschnitts (64) mit verringertem Durchmesser zwischen ca. 70 und ca. 80 mm beträgt.

8. Schubelement nach einem der vorhergehenden Ansprüche, worin der oder ein Höchstaußendurchmesser des Abschnitts (64) mit verringertem Außendurchmesser 8 mm ± 1 mm beträgt.

9. Schubelement nach einem der vorhergehenden Ansprüche, worin der oder ein Mindestaußendurchmesser des Abschnitts (64) mit verringertem Außendurchmesser 5 mm ± 1 mm beträgt.

10. Schubelement nach einem der vorhergehenden Ansprüche, worin der sich verjüngende Abschnitt zumindest eine der folgenden Eigenschaften aufweist: es ist eine lineare Verjüngung, eine gebogene Verjüngung oder eine runde Verjüngung.

11. Schubelement nach einem der vorhergehenden Ansprüche, worin der Abschnitt (64) mit verringertem Außendurchmesser zumindest teilweise zylindrisch ist.

12. Schubelement nach einem der vorhergehenden Ansprüche, worin das Schubelement (62) eine durch es hindurchgehende Bohrung umfasst.

13. Schubelement nach Anspruch 12, worin die Bohrung des Schubelements (62) eine glatte und im Wesentlichen gleichmäßige Innenwand aufweist.

14. Implantationsvorrichtung mit einem Schubelement (62) nach einem der vorhergehenden Ansprüche.

## Revendications

1. Organe (62) pour pousser conçu pour un dispositif de déploiement (10) ayant une forme tubulaire en général cylindrique avec un canal à l'intérieur permettant d'y recevoir un fil-guide, l'organe pour pousser (62) présentant une extrémité proximale et une extrémité distale, dans lequel l'extrémité distale de l'organe (62) pour pousser a une section de diamètre externe réduite (64) qui diminue graduellement vers son extrémité distale **caractérisée en ce qu'**un angle de diminution graduelle par rapport à l'axe longitudinal de l'organe pour pousser n'est pas plus de 2°.

2. Organe pour pousser selon la revendication 1, dans lequel l'organe (62) pour pousser a en majeure partie un diamètre externe sensiblement uniforme.

3. Organe pour pousser selon la revendication 1 ou 2, comprenant une tête (66) pour pousser entre la portion de diamètre externe réduite (64) et l'extrémité distale de l'organe pour pousser.

4. Organe pour pousser selon la revendication 3, dans lequel la tête (66) de l'organe pour pousser est munie d'un ou plusieurs alésages (170, 172) pour recevoir un fil de déclenchement et/ou libération du dispositif de déploiement.

5. Organe pour pousser selon l'une quelconque des revendications précédentes, dans lequel ladite section de diamètre externe réduite (64) s'étend sur une distance allant jusqu'à environ 200 mm.

6. Organe pour pousser selon l'une quelconque des revendications précédentes, dans lequel ladite section de diamètre externe réduite (64) s'étend sur une distance allant jusqu'à environ 150 mm.

7. Organe pour pousser selon l'une quelconque des revendications précédentes, dans lequel la longueur de la section de diamètre réduite (64) se situe entre environ 70 et environ 80 mm.

8. Organe pour pousser selon l'une quelconque des revendications précédentes, dans lequel le ou un diamètre externe maximum de la section de diamètre externe réduite (64) est de 8 mm ± 1 mm.

9. Organe pour pousser selon l'une quelconque de revendications précédentes, dans lequel le ou un diamètre externe minimum de la section de diamètre externe réduite (64) est de 5 mm ± 1 mm.

10. Organe pour pousser selon l'une quelconque des revendications précédentes, dans lequel la portion diminuant graduellement a au moins une des caractéristiques suivantes : elle est linéaire, courbe ou arrondie.

11. Organe pour pousser selon l'une quelconque des revendications précédentes, dans lequel la section de diamètre externe réduite (64) est au moins partiellement cylindrique.

12. Organe pour pousser selon l'une quelconque des revendications précédentes, dans lequel l'organe (62) pour pousser comprend un alésage traversant.

13. Organe pour pousser selon la revendication 12, dans lequel l'alésage de l'organe (62) pour pousser a une paroi interne lisse et sensiblement uniforme.

14. Dispositif de déploiement comprenant un organe (62) pour pousser selon l'une quelconque des revendications précédentes.
